# EUROPEAN PATENT APPLICATION

(11) **EP 4 488 687 A1**
(43) Date of publication of application: **08.01.2025**
(21) Application number: 24181655.2
(22) Date of filing: 12.06.2024
(51) Int. Cl.: G01N 33/68, A61K 39/00, A61P 1/00, C07K 16/28

(54) **METHOD FOR OBTAINING INFORMATION ON EFFICACY OF CHEMOTHERAPY FOR PATIENT WITH MEMBRANOUS NEPHROPATHY**

(30) Priority: 14.06.2023 JP 2023097806
(71) Applicant: SYSMEX CORPORATION, Kobe-shi Hyogo 651-0073 (JP)
(72) Inventor: Saruta, Yuko, Kobe-shi, Hyogo 651-0073 (JP); Hasegawa, Takehiro, Kobe-shi, Hyogo 651-0073 (JP); Zhao, Feng, Kobe-shi, Hyogo 651-0073 (JP)
(74) Representative: Hoffmann Eitle

(57) **Abstract**

Disclosed is a method for obtaining information on efficacy of chemotherapy for a patient with membranous nephropathy, the method comprising measuring CXCL13 in a biological sample collected from the patient with membranous nephropathy, in which the efficacy of chemotherapy for the patient with use of an anti-CD20 antibody drug is suggested by a measured result.

## Description

### FIELD OF THE INVENTION

The present invention relates to a method for obtaining information on efficacy of chemotherapy for a patient with membranous nephropathy. The present invention relates to a method for assisting determination of efficacy of chemotherapy for a patient with membranous nephropathy.

### BACKGROUND

Membranous nephropathy is a type of glomerulonephritis, and is most often diagnosed in patients with nephrotic syndrome. The nephrotic syndrome refers to a clinical condition typically characterized by edema, proteinuria, decreased plasma albumin, and increased blood cholesterol. In membranous nephropathy, a causative antigen and an autoantibody against the antigen are bound to produce an immune complex, and the complex then deposits on the epithelial side of the glomerular basement membrane to thicken the basement membrane. This degrades filtration function of the glomerulus, and causes chronic proteinuria and edema. Approximately 80% of membranous nephropathy is attributable to primary membranous nephropathy whose cause unknown (also referred to as idiopathic membranous nephropathy), and remaining approximately 20% is attributable to secondary membranous nephropathy typically associated with other disease (also referred to as secondary membranous nephropathy). Causes for the secondary membranous nephropathy include autoimmune diseases, infectious diseases, malignant tumors, and drugs. Recent report has described that M-type phospholipase A₂ receptor (PLA2R) expressed in glomerular epithelial cells is a causative antigen in approximately 70% of the primary membranous nephropathy. Currently, assay of anti-PLA2R antibody level in serum is used for risk classification of membranous nephropathy, and determination of treatment policy.

Prior treatment of membranous nephropathy has begun with single administration of a steroid, and, if the therapeutic effect is not sufficient, then optionally followed by combination therapy with use of the steroid and an immunosuppressive agent. It has recently reported that chemotherapy with use of rituximab, which is an anti-CD20 antibody drug, demonstrated a good therapeutic effect on patients with refractory nephrotic syndrome resulted from membranous nephropathy. Fernadez-Juarez G. et al, The STARMEN trial indicates that alternating treatment with corticosteroids and cyclophosphamide is superior to sequential treatment with tacrolimus and rituximab in primary membranous nephropathy, Kidney International, vol.99, pp.986-998, 2021, reported a trial (STARMEN trial) for verifying a hypothesis stating that the sequential therapy with tacrolimus and rituximab is superior to cyclical alternating treatment with corticosteroids and cyclophosphamide. In this trial, patients with persistent nephrotic syndrome due to primary membranous nephropathy were examined. Results revealed that the patients who enjoyed effects of the chemotherapy with rituximab was fewer than the patients who enjoyed effects of the alternating treatment with corticosteroid and cyclophosphamide. Approximately half of the patients were, however, found to enjoy effects of the chemotherapy with use of rituximab. The chemotherapy with use of the anti-CD20 antibody drug is therefore expected as a promising therapy for membranous nephropathy.

The chemotherapy with use of the anti-CD20 antibody drug is, however, not effective for all patients with membranous nephropathy, as described previously. The anti-CD20 antibody drug is expensive, and even would cause severe side effects. The anti-CD20 antibody drug is, therefore, necessarily administered only to the patients possibly demonstrating the effect. There is, however, no known means capable of predicting in advance the efficacy of chemotherapy with use of the anti-CD20 antibody drug for patients with membranous nephropathy. It is therefore an object of the present invention to provide a means capable of preliminarily determining whether or not chemotherapy with use of the anti-CD20 antibody drug is effective for a patient with membranous nephropathy.

### SUMMARY OF THE INVENTION

The present inventors have found CXCL13 (CXC chemokine ligand 13) as a biomarker associated with the efficacy of chemotherapy with use of an anti-CD20 antibody drug for patients with membranous nephropathy, and have completed the present invention. Hence, the following inventions [1] to [12] are provided.
[1] A method for obtaining information on efficacy of chemotherapy for a patient with membranous nephropathy, the method including measuring CXCL13 in a biological sample collected from the patient with membranous nephropathy, wherein the efficacy of chemotherapy for the patient with use of an anti-CD20 antibody drug is suggested by a result of the measuring.
[2] The method according to [1], wherein a measured value of CXCL13 below a threshold value suggests the efficacy of chemotherapy for the patient, meanwhile a measured value of CXCL13 equal to or above the threshold value suggests non-efficacy of chemotherapy for the patient.
[3] The method according to [1] or [2], wherein the anti-CD20 antibody drug is at least one selected from the group consisting of rituximab, obinutuzumab, ofatumumab, ibritumomab tiuxetan, tositumomab, ublituximab and pertuzumab.
[4] The method according to any one of [1] to [3], wherein the biological sample is a blood sample or urine.
[5] The method according to any one of [1] to [4], wherein the chemotherapy uses other drug, besides the anti-CD20 antibody drug.
[6] The method according to [5], wherein the other drug is at least one selected from the group consisting of immunosuppressant and steroid.
[7] The method according to [6], wherein the immunosuppressant is a calcineurin inhibitor.
[8] The method according to [7], wherein the calcineurin inhibitor is at least one selected from the group consisting of tacrolimus, pimecrolimus, cyclosporine, and voclosporin.
[9] The method according to [6], wherein the steroid is corticosteroid.
[10] A method for assisting determination of efficacy of chemotherapy for a patient with membranous nephropathy, the method including:
   measuring CXCL13 in a biological sample collected from the patient with membranous nephropathy; and
   determining, from a result of the measuring, the efficacy of chemotherapy for the patient with use of an anti-CD20 antibody drug.
[11] The method according to [10], wherein the chemotherapy is determined to be effective on the patient if a measured value of CXCL13 is below a threshold value, meanwhile determined to be not effective on the patient if a measured value of CXCL13 is equal to or above the threshold value.
[12] A reagent kit for use in the method according to any one of [1] to [11], the reagent kit containing a reagent that contains a substance capable of specifically binding to CXCL13.

The present invention can determine whether or not the chemotherapy with use of an anti-CD 20 antibody drug is effective for a patient with membranous nephropathy.

### BRIEF DESCRIPTION OF THE DRAWINGS

Fig. 1 is a drawing illustrating an exemplary appearance of a reagent kit.
Fig. 2 is a boxplot illustrating CXCL13 levels in urine samples (uCXCL13) from a response group and a non-response group in chemotherapy.
Fig. 3 is a boxplot illustrating IL-17 levels in urine samples (uIL17) from a response group and a non-response group in chemotherapy.
Fig. 4 is a boxplot illustrating TWEAK levels in urine samples (uTWEAK) from a response group and a non-response group in chemotherapy.
Fig. 5 is a boxplot illustrating GDF15 levels in urine samples (uGDF15) from a response group and a non-response group in chemotherapy.
Fig. 6 is a boxplot illustrating TNF-α levels in urine samples (uTNF-α) from a response group and a non-response group in chemotherapy.
Fig. 7 is a boxplot illustrating serum CXCL13 levels of a response group and a non-response group in chemotherapy.
Fig. 8 is a boxplot illustrating serum IL-17 levels of a response group and a non-response group in chemotherapy.
Fig. 9 is a boxplot illustrating serum TWEAK levels of a response group and a non-response group in chemotherapy.
Fig. 10 is a boxplot illustrating serum GDF-15 levels of a response group and a non-response group in chemotherapy.
Fig. 11 is a boxplot illustrating serum TNF-α levels of a response group and a non-response group in chemotherapy.
Fig. 12 is a boxplot illustrating serum MIG levels of a response group and a non-response group in chemotherapy.
Fig. 13 is a boxplot illustrating serum CCL20 levels of a response group and a non-response group in chemotherapy.
Fig. 14 is a boxplot illustrating serum anti-PLA2R antibody levels of a response group and a non-response group in chemotherapy.

### DETAILED DESCRIPTION OF THE PREFERRED EMBODIMENTS

In the method for acquiring information on the efficacy of chemotherapy for patients with membranous nephropathy, CXCL13 in a biological sample collected from a patient with membranous nephropathy is measured.

As used herein, the term "patient with membranous nephropathy" includes both patient diagnosed with membranous nephropathy and patient suspected of membranous nephropathy. The membranous nephropathy may be either primary membranous nephropathy or secondary membranous nephropathy. The "patient with membranous nephropathy" is preferably a patient with membranous nephropathy who has not yet undergone chemotherapy with use of an anti-CD20 antibody drug. The "patient with membranous nephropathy" is more preferably a patient with membranous nephropathy who is subject to immunosuppressive therapy and has not yet undergone chemotherapy with use of an anti-CD20 antibody drug. The immunosuppressive therapy means treatment with use of an immunosuppressant.

The membranous nephropathy is determined by diagnosing related symptom, and by definitive diagnosis such as serum anti-PLA2R antibody test and kidney biopsy. The diagnosis of the related symptom is exemplified by X-ray or CT scan for diagnosing the presence or absence of sarcoidosis; medication history typically with non-steroidal anti-inflammatory drug (NSAID), gold drug or penicylamine; anti-nuclear antibody test; malignant tumor screening; renal echo; diagnosis of hepatitis B, hepatitis C, HIV, and treponema infection; history of diseases such as thyroid disease and systemic disease; and physical findings such as skin, joint and so forth.

Chemotherapy with use of anti-CD20 antibody drug refers to a method of treating disease with medication of at least anti-CD 20 antibody drug. The anti-CD20 antibody drug refers to a medicine that contains an antibody capable of specifically binding to CD20, and of demonstrating cytotoxic and depleting effects on CD20 positive cell. CD20 has been known to be expressed specifically on the surface of differentiating B cell, and B cell lymphoma cell. Antibody contained in the anti-CD20 antibody drug is preferably chimeric (human/mouse) monoclonal antibody or fully human monoclonal antibody. The antibody contained in the anti-CD20 antibody drug may be labeled with a pharmaceutically acceptable radioactive substance such as iodine 131. The anti-CD20 antibody drug per se has been known, and is exemplified by rituximab, obinutuzumab, ofatumumab, ibritumomab tiuxetan, tositumomab, ublituximab and pertuzumab.

The chemotherapy with use of the anti-CD20 antibody drug may use some other drug, besides the anti-CD20 antibody drug. The other drug is exemplified by immunosuppressant and steroid. The immunosuppressant is exemplified by calcineurin inhibitor, alkylating agent, purine antimetabolite, and an mTOR inhibitor. The calcineurin inhibitor is exemplified by tacrolimus, pimecrolimus, cyclosporin, and voclosporin. The alkylating agent is exemplified by cyclophosphamide and chlorambucil. The purine antimetabolite is exemplified by mizoribine, mycophenolate mofetil, and azathioprine. The mTOR inhibitor is exemplified by everolimus, sirolimus, and temsirolimus. Among them, the calcineurin inhibitor is preferred. The steroid is preferably corticosteroid, and is exemplified by prednisolone.

CXCL13 is a type of chemokine, and is also referred to BCA-1 (B cell attracting chemokine 1), or BLC (B lymphocyte chemoattractant). CXCL13 is known as a protein ligand for CXCR5 receptor expressed on the surfaces ofB cell and follicular T cell. Amino acid sequence of CXCL13 per se has been known, which is disclosed for example in a known database at NCBI (National Center for Biotechnology Information).

The biological sample is not particularly limited as long as it is a sample that contains CXCL13. Examples of such a biological sample include blood samples, urine, cerebrospinal fluid, pleural effusion, ascites, and lymph. The blood sample includes blood (whole blood) collected from the subject, and plasma or serum prepared from the blood. Any insoluble impurity such as cell, if contained in the biological sample, may be removed therefrom by a known technique such as centrifugation or filtration. The biological sample may optionally be diluted with an appropriate aqueous medium. Such aqueous medium is not specially limited as long as it does not interfere with measurements described later, and is exemplified by water, saline and buffer. The buffer is not specially limited as long as it can demonstrate buffering action at pH around neutral (pH 6 or higher and pH 8 or lower, for example). Such buffer is exemplified by Good's buffers such as HEPES, MES and PIPES; phosphate-buffered saline (PBS); and Tris-buffered saline (TBS).

In this specification, "measuring CXCL13" includes determining the amount or level of CXCL13, and acquiring information that reflects the amount or level of CXCL13. The "information that reflects the amount or level of CXCL13" means an index that varies corresponding to the amount or level of CXCL13 in a biological sample or a measurement sample prepared from the biological sample. Such index preferably represents an optical change which is visually recognizable or mechanically measurable. The index of the optical change is exemplified by emission intensity, fluorescence intensity, absorbance, turbidity, and color density.

The information that reflects the amount or level of CXCL13 may be expressed qualitatively, quantitatively, or semi-quantitatively. The qualitatively expressed information relates to presence or absence of CXCL13. The quantitatively expressed information relates to numerical information such as a numerical value obtained with a measuring instrument (hereinafter, also referred to as "raw data"), and a value calculated from such numerical value. The value calculated from the raw data is exemplified by a value obtainable by subtracting a measured value of a negative control or a background value, from the raw data. The amount or level of CXCL13 may be determined with reference to the quantitative information. The semi-quantitatively expressed information relates to graded presentation of the amount or level of CXCL13 typically with word, number (ranking), or color. The word usable herein may be "not detectable", "few", "medium" of "much".

In the present specification, the "measured result of CXCL13" includes a value obtained by measuring CXCL13, information, and a combination thereof. The measured result of CXCL13 preferably relates to quantitative information that reflects the amount or level of CXCL13, and/or a value that represents the amount or level of CXCL13 determined from the quantitative information. Hereinafter, the quantitative information that reflects the amount or level of CXCL13, and/or the value that represents the amount or level of CXCL13 will be also referred to as "measured value of CXCL13".

Method for measuring CXCL13 is not particularly limited as long as the method can acquire information that reflects the amount or level of CXCL13 in the biological sample or the measurement sample prepared from the biological sample. Examples of such method include capturing of CXCL13 with use of a substance capable of specifically binding to CXCL13. CXCL13 in the biological sample may be measured by a known method, by detecting CXCL13 captured by such substance.

The substance capable of specifically binding to CXCL13 is exemplified by antibody, aptamer, and receptor protein. Among them, antibody is particularly preferred. The antibody against CXCL13 has been known, and is commercially available. The antibody against CXCL13 is not particularly limited as long as it can specifically bind to CXCL13. Such antibody may be any of monoclonal antibody, polyclonal antibody, and fragments thereof (Fab, F(ab')₂, Fab', for example). Alternatively, a commercially available antibody may be used.

Method for measuring CXCL13 is properly selectable from known immunoassay methods without special limitation. Such method is exemplified by enzyme-linked immunosorbent assay (ELISA), and western blotting. Among them, ELISA is preferred. ELISA of any type, including sandwich method, competitive method, direct method or indirect method, is applicable. The sandwich method is particularly preferred. Paragraphs below will describe an exemplary process of measuring CXCL13 in a biological sample, by sandwich ELISA.

First, a complex is formed on a solid phase, wherein the complex is composed of CXCL13, an antibody for capturing CXCL13 (also referred to as "capture antibody", hereinafter), and an antibody for detecting CXCL13 (also referred to as "detection antibody", hereinafter). The complex may be formed by mixing a biological sample that presumably contains CXCL13, the capture antibody, and the detection antibody. The complex may be formed on the solid phase, by contacting a solution that contains the complex, with the solid phase capable of capturing thereon the capture antibody Alternatively, the solid phase having the capture antibody preliminarily immobilized thereon may be used. That is, the complex may be formed on the solid phase, by contacting the solid phase having the capture antibody immobilized thereon, the biological sample, and the detection antibody. Note that the capture antibody and the detection antibody, if both being monoclonal antibodies, preferably have different epitopes.

The solid phase may only be an insoluble carrier on which the capture antibody may be immobilized. Mode of immobilization of the capture antibody on the solid phase is not specially limited. For example, the capture antibody and the solid phase may be directly bound, or the capture antibody and the solid phase may be indirectly bound while placing some other substance in between. The direct binding is exemplified by physical adsorption. The indirect binding is exemplified by binding making use of combination of biotins and avidins. The capture body may be immobilized on the solid phase, via a bond between the biotins and avidins, with use of the capture body preliminarily modified with biotins, and the solid phase having avidins preliminarily bound thereto.

In this specification, the term "biotins" encompasses biotin and biotin analog. The biotin analog is exemplified by desthiobiotin and biocytin. In this specification, the term "avidins" encompasses avidin and avidin analog. The avidin analog is exemplified by streptavidin, avidin-like protein derived from Paecilomyces tenuipes (Tamavidin (registered trademark)), bradavidin, and rhizavidin.

Material for composing the solid phase is selectable, without special limitation, typically from organic polymer compound, inorganic compound, and biopolymer. The organic polymer compound is exemplified by latex, polystyrene and polypropylene. The inorganic compound is exemplified by magnetic substance (iron oxide, chromium oxide, ferrite, etc.), silica, alumina and glass. The biopolymer is exemplified by insoluble agarose, insoluble dextran, gelatin and cellulose. Two or more of them may be used in combination. The solid phase may have any form not specifically limited, and is exemplified by particle, microplate, membrane, microtube and test tube. Among them, particle is preferred, and magnetic particle is particularly preferred.

B/F (bound/free) separation for removing any free component that remains unreacted without forming the complex may take place between formation of the complex and detection of the complex. The free component that remains unreacted refers to a component that does not constitute a complex. This is exemplified by capture antibody and the detection antibody which remained unbound with CXCL13. Technique for the B/F separation is not specially limited, and may be conducted, in an exemplary case where the solid phase is a particle, by centrifugation so as to collect only the solid phase having the complex bound thereon. With the solid phase given as a container such as a microplate or a microtube, the B/F separation may rely upon removal of a liquid that contains the unreacted free component. With the solid phase given as a magnetic particle, the B/F separation may rely upon removal of a liquid that contains the unreacted free component, under suction through a nozzle, while magnetically restraining the magnetic particle with use of a magnet. The method is preferred from the viewpoint of automatization. After removing the unreacted free components, the solid phase having the complex bound thereon may be washed with suitable aqueous medium such as PBS.

CXCL13 in the biological sample may be measured by detecting, with a known method, the complex formed on the solid phase. For an exemplary case where the antibody labeled with a labeling substance is used as the detection antibody, a signal ascribed to the labeling substance is generated from the detection antibody contained in the complex on the solid phase. The complex on the solid phase is detected by detecting the signal. Since the complex contains CXCL13 captured from the biological sample with use of the capture antibody, so that CXCL13 in the biological sample is measured by detecting the complex. In an alternative case where a labeled secondary antibody for the detection antibody is used, CXCL13 in the biological sample may be measured in the same manner.

In yet another example of measuring CXCL13 with use of an antibody, an immune complex transfer method described in JP H01-254868 A may be used.

The labeling substance is not particularly limited as long as a detectable signal is generated. The labeling substance may generate a signal by itself (also referred to as "signal generating substance", hereinafter), or may catalyze reaction of some other substance to generate the signal. The signal generating substance is exemplified by fluorescent substance, and radioisotope. The substance that catalyzes reaction of some other substance to generate the signal is exemplified by enzyme. The enzyme is exemplified by alkaline phosphatase, peroxidase, β-galactosidase, and luciferase. The fluorescent substance is exemplified by fluorescent dyes such as fluorescein isothiocyanate (FITC), rhodamine, and Alexa Fluor (registered trademark); and fluorescent protein such as GFP. The radioisotope is exemplified by ¹²⁵I, ¹⁴C and ³²P. The labeling substance is preferably enzyme, among which alkaline phosphatase and peroxidase are particularly preferred.

The method for detecting signal per se has been already known in the art. In this embodiment, it suffices to properly select a measurement method suited to the type of signal attributable to the labeling substance. In an exemplary case where the labeling substance is an enzyme, a signal such as light or color, resulted from a reaction of the enzyme with a corresponded substrate, may be measured with use of a known instrument such as a spectrophotometer.

The substrate for the enzyme is properly selectable from known substrates, depending on the type of enzyme. In an exemplary case where alkaline phosphatase is used as the enzyme, the substrate is exemplified by chemiluminescent substrates such as CDP-Star (registered trademark) (disodium 4-chloro-3-(methoxyspiro[1,2-dioxetane-3,2'-(5'-chloro)tricyclo[3.3.1.1^{3,7}]decane]-4-yl)phenylphosphate), and CSPD (registered trademark) (disodium 3-(4-methoxyspiro[1,2-dioxetane-3,2-(5'-chloro)tricyclo[3.3.1.1^{3,7}]decane]-4-yl) phenylphosphate); and chromogenic substrate such as 5-bromo-4-chloro-3-indolylphosphate (BCIP), disodium 5-bromo-6-chloro-indolylphosphate, and p-nitrophenyl phosphate. In an exemplary case where peroxidase is used as the enzyme, the substrate is exemplified by chemiluminescent substrates such as luminol and derivatives thereof; and chromogenic substrates such as ammonium 2,2'-azinobis(3-ethylbenzothiazoline-6-sulfonate) (ABTS), 1,2-phenylenediamine (OPD), and 3,3',5,5'-tetramethylbenzidine (TMB).

In a case where the labeling substance is a radioisotope, radiation may be measured as the signal by using a known instrument such as a scintillation counter. In a case where the labeling substance is a fluorescent substance, fluorescence may be measured as the signal by using a known instrument such as a fluorescence microplate reader. Excitation wavelength and fluorescence wavelength may properly be determined depending on the type of fluorescent substance employed.

Results of signal detection may be utilized as the measured result of CXCL13. When quantifying the signal intensity, the measured value of the signal intensity per se, or a value acquired from the measured value, may be used as the measured value of CXCL13. The value acquired from the measured value of the signal intensity is exemplified by a value obtainable by subtracting a measured value of a negative control or a background value, from the measured value of the signal intensity. The measured value of the signal intensity may alternatively be fitted on an analytical curve, to determine the amount or level of CXCL13. The negative control sample can be appropriately selected, which may typically be a biological sample obtained from a healthy person.

CXCL13 contained in the biological sample may be measured by sandwich ELISA that uses the capture antibody immobilized on the magnetic particle, and the detection antibody labeled with the labeling substance. The measurement in this case may use a commercially available fully automated immunoassay system. This sort of fully automated immunoassay system is exemplified by those in HISCL (registered trademark) Series from Sysmex Corporation.

The measured result of CXCL13 can serve as an index of the efficacy of chemotherapy with use of an anti-CD20 antibody drug for a patient with membranous nephropathy. For example, as will be explained later in Examples, a response group who responded to rituximab was found to have a CXCL13 level in the biological sample, significantly lower than that in a patient group who did not respond to rituximab. The measured result of CXCL13 can therefore be acquired as information regarding the efficacy of chemotherapy with use of an anti-CD20 antibody drug for the patients with membranous nephropathy. For example, a measured value of CXCL13 below a threshold value can suggest efficacy of chemotherapy with use of anti-CD20 antibody drug in a patient with membranous nephropathy. A measured value of CXCL13 equal to or above the threshold value can suggest nonefficacy of chemotherapy with use of anti-CD20 antibody drug in a patient with membranous nephropathy.

Alternatively, a measured value of CXCL13 equal to or below the threshold value can suggest efficacy of chemotherapy with use of anti-CD20 antibody drug in a patient with membranous nephropathy. A measured value of CXCL13 above the threshold value can suggest nonefficacy of chemotherapy with use of anti-CD20 antibody drug in a patient with membranous nephropathy.

The threshold value for the measured value of CXCL13 may be properly determined, without special limitation. The threshold value may be set, typically as below. First, biological samples are collected from a plurality of patients with membranous nephropathy who responded to chemotherapy with use of the anti-CD20 antibody drug (response group), and from a plurality of patients with membranous nephropathy who did not respond to the chemotherapy (non-response group), and CXCL13 is measured to obtain measured values of CXCL13. A value that can most accurately distinguish the response group and the non-response group is then determined as the threshold value to be set. The threshold value is preferably determined, typically considering sensitivity, specificity, positive predictive value, and negative predictive value.

The present invention may include determining, from the measured result, the efficacy of chemotherapy with use of an anti-CD20 antibody drug for the patient with membranous nephropathy. The determination preferably stands on a result of comparison between the measured value of CXCL13 and the threshold value. For example, a measured value of CXCL13 below the threshold value can determine that the chemotherapy with use of anti-CD20 antibody drug in a patient with membranous nephropathy makes effect. A measured value of CXCL13 equal to or above the threshold value can determine that the chemotherapy with use of anti-CD20 antibody drug in a patient with membranous nephropathy does not make effect. Alternatively, a measured value of CXCL13 equal to or below the threshold value can determine that the chemotherapy with use of anti-CD20 antibody drug in a patient with membranous nephropathy makes effect. A measured value of CXCL13 above the threshold value can determine that the chemotherapy with use of anti-CD20 antibody drug in a patient with membranous nephropathy does not make effect. Details of the patient with membranous nephropathy, the chemotherapy with use of the anti-CD20 antibody drug, the biological sample, the measurement of CXCL13, and the threshold are as described above.

The present invention also includes a method for treating a patient with membranous nephropathy, according to a result of determination. The method of treatment includes: measuring CXCL13 in a biological sample collected from the patient with membranous nephropathy; determining, from a measured result, the efficacy of chemotherapy for the patient with use of an anti-CD20 antibody drug; and subjecting the patient to the chemotherapy with use of the anti-CD20 antibody drug, if the chemotherapy is determined to be effective. In the determination, a measured value of CXCL13 below the threshold value can determine that the chemotherapy with use of anti-CD20 antibody drug in a patient with membranous nephropathy makes effect. Alternatively, a measured value of CXCL13 equal to or below the threshold value can determine that the chemotherapy with use of anti-CD20 antibody drug in a patient with membranous nephropathy makes effect. Details of the patient with membranous nephropathy, the chemotherapy with use of the anti-CD20 antibody drug, the biological sample, the measurement of CXCL13, and the threshold are as described above.

In the chemotherapy, an effective dose of the anti-CD20 antibody drug is preferably administered to the patient. Other immunosuppressant may further be administered, besides the anti-CD20 antibody drug. The effective dose may properly be determined, typically following therapeutic guidelines for membranous nephropathy.

The present invention also encompasses a kit (referred to as "reagent kit", hereinafter) used for a method for acquiring information on efficacy of chemotherapy for patient with membranous nephropathy, or for a method for assisting determination of efficacy of chemotherapy for patient with membranous nephropathy. The reagent kit contains a reagent that contains a substance capable of specifically binding to CXCL13. Details of the substance capable of specifically binding to CXCL13 are as described above.

The reagent that contains a substance capable of specifically binding to CXCL13 preferably consists of a combination of a reagent that contains a capture antibody for CXCL13, and a reagent that contains a detection antibody for CXCL13. The reagent kit contains a reagent that contains a capture antibody for CXCL13, and a reagent that contains a detection antibody for CXCL13. The detection antibody may be labeled with a labeling substance. The reagent kit, containing an enzyme as the labeling substance, may contain a substrate for the enzyme. Details of the capture antibody, the detection antibody, the labeling substance, and the substrate are as described above. The capture antibody, detection antibody, labeling substance, and substrate may have any form including solid (powder, crystal, freeze-dried product, etc.), or liquid (solution, suspension, emulsions, etc.), without special limitation.

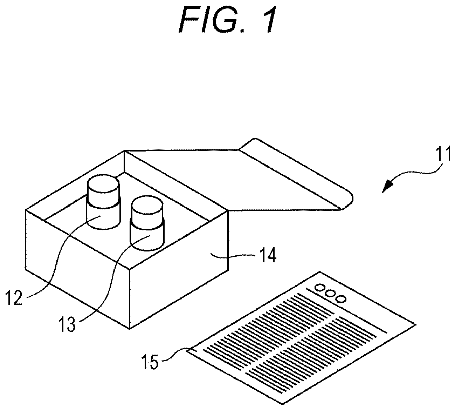

The reagent kit may be provided to the user, with the individual reagents filled in vials and packaged in a box. The box may include a package insert. The package insert may typically describe a composition of the reagent kit, a method of use, and relationship between measured result obtained with use of the reagent kit and clinical conditions of patients with membranous nephropathy. An exemplary reagent kit is illustrated in Fig. 1. The reagent kit illustrated in Fig. 1 contains a reagent that contains a capture antibody for CXCL13, and a reagent that contains a detection antibody for CXCL13, but not limited thereto. Referring now to Fig. 1, reference numeral 11 denotes the reagent kit, reference numeral 12 denotes a first vial filled with the reagent that contains the capture antibody for CXCL13, reference numeral 13 denotes a second vial filled with the reagent that contains the detection antibody for CXCL13, reference numeral 14 denotes a packaging box, and reference numeral 15 denotes a package insert. In this example, the reagent kit may further contain a solid phase for immobilizing the capture antibody for CXCL13. Details of the solid phase are as described above.

The reagent kit of this embodiment may further contain a calibrator for quantifying CXCL13. The calibrator for quantifying CXCL13 is typically at least one buffers that contains a predetermined concentration of a recombinant protein of CXCL13. Only one kind of, or a plurality of kinds of the buffer that contains a predetermined concentration of CXCL13 may be used. The plurality of kinds of the buffer, when used, are preferably prepared to contain different concentrations of CXCL13, typically as a result of serial dilution. The calibrator for quantifying CXCL13 may further contain a buffer solution free of CXCL13 (negative control).

The present invention includes use of a reagent for producing a reagent kit used in a method for assisting determination of the efficacy of chemotherapy for a patient with membranous nephropathy. Details of the reagent are as described above.

The present invention will be further detailed below, referring to Examples, to which the present invention is by no means limited.

### EXAMPLES

### Example 1

### (1) Biological Samples

Biological samples used herein were urine samples and blood samples from 14 patients of primary membranous nephropathy who were subjected to treatment with rituximab and tacrolimus in the STARMEN test (see Femadez-Juarez G. et al, The STARMEN trial indicates that alternating treatment with corticosteroids and cyclophosphamide is superior to sequential treatment with tacrolimus and rituximab in primary membranous nephropathy, Kidney International, vol.99, pp.986-998, 2021). These patients had not undergone chemotherapy with rituximab and tacrolimus, at the time the urine and blood were collected. The urine sample of each patient was prepared by diluting urine, collected during the second urination in the morning, four times with PBS (containing 1% bovine serum albumin (BSA), 0.05% sodium azide and 50 mM EDTA). The blood sample was serum prepared from blood collected from each patient by a usual method. These samples were obtained from REDinREN Biobank (Universidad de Alcala, Facultad de Medicina; address: Carretera Madrid-Barcelona, Km. 33.600, Alcala de Henares, Madrid Spain; representative: Laura Calleros Basilio).

### (2) Patient Information

The aforementioned patients were adults diagnosed by biopsy as primary membranous nephropathy, having underwent an observation period of 6 months or longer. eGFR per 1.73 m² was 45 mL/min or higher (≥ 45 mL/min/1.73 m²). Proteinuria value was higher than 4 g/24 hours (> 4 g/24 h), indicating nephrotic syndrome. The proteinuria value was not found to decrease more than 50% throughout the observation period. During the observation period, the serum albumin level was found to be 3.5 g/dL or below (<_ 3.5 g/dL), indicating hypoalbuminemia. The aforementioned patients underwent standard therapy (therapy with angiotensin-converting enzyme inhibitor/angiotensin receptor blocker) for at least two months, and blood pressure control (≤ 150/90 mmHg) for at least three months.

### (3) Chemotherapy with Rituximab and Tacrolimus

Tacrolimus (0.05 mg/kg/day) was orally administered to the aforementioned patients for six months. The target blood concentration was 5 to 7 ng/mL. On Day 180 from the start of administration, rituximab (1 g) was intravenously administered, while reducing tacrolimus by 25% per month. Therapy was terminated after nine months. Tacrolimus dose was reduced for the patients with renal dysfunction. Methylprednisolone (100 mg), acetaminophen (1 g), and diphenhydramine (50 mg) were administered, for reducing possible side effects of rituximab. Also trimethoprim/sulfamethoxazole (160/800 mg) was orally administered three times a week, throughout the treatment period.

### (4) Classification of Response Group and Non-Response Group of Chemotherapy

Patients who reached complete or partial remission of membranous nephropathy, after the chemotherapy with use of rituximab and tacrolimus, were classified into the response group. Five patients were classified into the response group. The complete remission was defined if proteinuria decreased to fall within a 0.3 g/24 hours range (<_ 0.3 g/24 h) from the baseline, while maintaining the renal function (eGFR per 1.73 m² was 45 mL/min or higher). The partial remission was defined if proteinuria decreased from the baseline by a degree exceeding 50%, or proteinuria is kept below 3.5 g per 24 hours (< 3.5 g/24 h), while maintaining the renal function (eGFR per 1.73 m² was 45 mL/min or higher). The patients below were classified into the non-response group. Patients who demonstrated less than 50% reduction of proteinuria from the baseline, and relapsed nephrotic syndrome. Nine patients were classified into the non-response group. The patients who relapsed nephrotic syndrome were defined as those who had previously reached a partial or complete remission, but relapsed a proteinuria level of 3.5 g/24 hours or more, and demonstrated a proteinuria level increased by at least 50% or more from the minimum, when diagnosed at the hospital three times in a row.

### (5) Measurement of Biomarkers

CXCL13, IL-17, TWEAK, GDF15 and TNF-α in the urine samples of the individual patients were measured. Also CXCL13, IL-17, TWEAK, GDF15, TNF-α, MIG (also referred to as CXCL9), CCL20 (also referred to as MIP-3α), and anti-PLA2R antibody in the blood samples of the individual patients were measured. IL-17, TWEAK, GDF15, TNF-α, MIG and CCL20 are biomarkers having been reported to be associated with glomerulonephritis and/or autoimmune diseases. Anti-PLA2R antibody has been known as a causative antigen for primary membranous nephropathy. The samples were measured with an fully automated immunoassay system HISCL-5000 (Sysmex Corporation). GDF15 was measured with use of an ELISA reagent purchased from R&D Systems. The anti-PLA2R antibody was measured with use of an ELISA reagent purchased from Euroimmun. The biomarkers other than GDF15 and the anti-PLA2R antibody were measured with use of R1 to R5 reagents below. R1 reagent used for measuring the biomarkers other than TNF-α was a capture reagent that contains an antibody capable of specifically binding to each biomarker other than TNF-α. R1 reagent used for the measurement of TNF-α was a MES buffer described later. R2 reagent used for measuring the biomarkers other than TNF-α was a reagent that contains a magnetic particle as a solid phase. R2 reagent used for measuring TNF-α was a reagent that contains a magnetic particle which is a solid phase having immobilized thereon an antibody capable of specifically binding to TNF-α. R3 reagent was a detection reagent that contains an ALP-labeled antibody capable of specifically binding to each biomarker. R4 reagent was a measurement buffer, and R5 reagent was a reagent that contains a chemiluminescent substrate of ALP. R4 and R5 reagents were commonly used for measuring the individual biomarkers.

### (5.1) Reagent for CXCL13 Measurement

### • R1 Reagent

A biotin-labeled anti-CXCL13 monoclonal antibody (Invitrogen) was dissolved in a buffer, to prepare R1 reagent. R1 reagent for measuring CXCL13 in the urine samples employed 100 mM triethanolamine (pH 7.4) (containing 150 mM NaCl, 1% BSA, and 0.5% casein) as the buffer. R1 reagent for measuring CXCL13 in the blood samples employed 100 mM HEPES (pH 7.4) (containing 550 mM NaBr, 0.15 mM EDTA 2Na and 25 mM EGTA) as the buffer.

### • R2 Reagent

A magnetic particle having streptavidin immobilized on the surface (also referred to as "STA-bound magnetic particle" hereinafter; average particle size: 2 µm; 2.9 to 3.5 mg streptavidin bound per 1 g of magnetic particle) was washed 3 times with 10 mM HEPES (pH 7.5). The washed STA-bound magnetic particle was added to 10 mM HEPES (pH 7.5) so as to adjust the streptavidin concentration to 18 to 22 µg/ml (the STA-bound magnetic particle concentration adjusted to 0.48 to 0.52 mg/ml), to obtain R2 reagent.

### • R3 Reagent

An anti-CXCL13 monoclonal antibody (BioLegend Inc.), derived from a clone different from that of the antibody used in R1 reagent, was labeled with ALP by an usual method. The obtained ALP-labeled anti-CXCL13 antibody was dissolved in 100 mM triethanolamine (pH 7.4) (containing 150 mM NaCl and 1% BSA), to prepare R3 reagent.

### • R4 Reagent and R5 Reagent

R4 reagent used herein was HISCL R4 reagent (from Sysmex Corporation), which is a measurement buffer. R5 reagent used herein was HISCL R5 reagent (Sysmex Corporation) that contains CDP-Star (registered trademark) (from Applied Biosystems), which is a chemiluminescent substrate of ALP.

### (5.2) Reagent for IL-17 Measurement

### • R1 Reagent

A biotin-labeled anti-IL-17 monoclonal antibody (Invitrogen) was dissolved in 100 mM triethanolamine (pH 7.4) (containing 150 mM NaCl, 1% BSA, and 0.5% casein), to prepare R1 reagent.

### • R3 Reagent

An ALP-labeled anti-IL-17 monoclonal antibody (Invitrogen), derived from a clone different from that of the antibody used in R1 reagent, was dissolved in 100 mM triethanolamine (pH 7.4) (containing 150 mM NaCl and 1% BSA), to prepare an R3 reagent.

### (5.3) Reagents for TWEAK Measurement

### • R1 Reagent

An anti-human TWEAK monoclonal antibody (R&D Systems, Inc.) was labeled with biotin by an usual method. The obtained biotin-labeled anti-TWEAK antibody (Invitrogen) was dissolved in 100 mM triethanolamine (pH 7.4) (containing 150 mM NaCl, 1% casein, 10 mM EDTA, and 10 mM EGTA), to prepare R1 reagent.

### • R3 Reagent

An anti-TWEAK monoclonal antibody (Abcam Ltd.), derived from a clone different from that of the antibody used in R1 reagent, was labeled with ALP by an usual method. The obtained ALP-labeled anti-TWEAK antibody was dissolved in 100 mM triethanolamine (pH 7.4) (containing 150 mM NaCl and 1% BSA), to prepare an R3 reagent.

### (5.4) Reagents for TNF-α Measurement

### • R1 Reagent

As R1 reagent, 100 mM MES (pH 6.5) (containing 150 mM NaCl and 2% BSA) was prepared.

### • R2 Reagent

An anti-TNF-α monoclonal antibody (Bio-Techne Corporation) was labeled with biotin by an usual method. The obtained biotin-labeled anti-TNF-α antibody was adsorbed to a magnetic particle to be primed, and dissolved in 100 mM MES (pH 6.5) (containing 150 mM NaCl and 2% BSA), to prepare an R2 reagent.

### • R3 Reagent

An anti-TNF-α monoclonal antibody (Biotechnology Inc.), derived from a clone different from that of the antibody used in R2 reagent, was labeled with ALP by an usual method. The obtained ALP-labeled anti-TNF-α antibody was dissolved in 100 mM MES (pH 6.5) (containing 150 mM NaCl and 2% BSA), to prepare R3 reagent.

### (5.5) Reagents for MIG Measurement

### • R1 Reagent

An anti-MIG monoclonal antibody (RANDOX Laboratories Ltd.) was digested with pepsin or IdeS protease by an usual method, to obtain a Fab fragment. The Fab fragment was labeled with biotin by an usual method. The obtained biotin-labeled Fab fragment was dissolved in 100 mM triethanolamine (pH 7.4) (containing 150 mM NaCl, 1% BSA and 0.5% casein), to prepare R1 reagent.

### • R3 Reagent

An anti-MIG monoclonal antibody (from RANDOX), derived from a clone different from that of the antibody used in R1 reagent, was digested with pepsin or IdeS protease, to obtain a Fab fragment. The Fab fragment was labeled with ALP by an usual method. The obtained ALP-labeled Fab fragment was dissolved in 100 mM triethanolamine (pH 7.4) (containing 150 mM NaCl, 1% CHAPS, 1% BSA and 0.5% casein), to prepare R3 reagent.

### (5.6) Reagents for CCL20 Measurement

### • R1 Reagent

An anti-MIP-3α monoclonal antibody (Abcam Ltd.) was used as R1 reagent for measuring CCL20 in the urine samples. The antibody was labeled with biotin by an usual method, and the obtained biotin-labeled antibody was dissolved in 100 mM triethanolamine (pH 7.4) (containing 150 mM NaCl, 25 mM EDTA, 25 mM EGTA, and 0.1% Tween (trademark) 20), to prepare R1 reagent. An anti-human CCL20 (MIP-3α) monoclonal antibody (BioLegend Inc.) was used as R1 reagent for measuring CCL20 in the blood samples. The antibody was labeled with biotin by an usual method, and the obtained biotin-labeled antibody was dissolved in 100 mM triethanolamine (pH 7.4) (containing 150 mM NaCl, 25 mM EDTA, 25 mMEGTA, and 0.1% Tween (trademark) 20), to prepare R1 reagent.

### • R3 Reagent

An anti-human CCL20 (MIP-3α) monoclonal antibody (BioLegend Inc.), derived from a clone different from that of the antibody used in R1 reagent, was labeled with ALP by an usual method. The obtained ALP-labeled antibody was dissolved in 100 mM triethanolamine (pH 7.4) (containing 150 mM NaCl, 1% BSA, and 0.5% casein), to prepare R3 reagent.

Measurement procedures with use of HISCL-5000 were as follows. For biomarkers other than TNF-α, the urine samples were diluted 2 times to 4 times with a sample buffer (PBS containing 1% BSA and 50 mM EDTA (pH 7.4) [CXCL13]; PBS containing 1% casein (pH 7.4) [TWEAK]; and PBS containing 1% BSA (pH 7.4) [IL-17]), to prepare the measurement samples. Each measurement sample was mixed with R1 reagent, to which R2 reagent was then added. The magnetic particle in the resultant mixed liquid was magnetically collected to remove the supernatant, and the magnetic particle was washed by adding HISCL washing solution. The supernatant was removed, and R3 reagent was added to the magnetic particle, followed by mixing. The magnetic particle in the resultant mixed liquid was magnetically collected to remove the supernatant, and the magnetic particle was washed by adding HISCL washing solution. The supernatant was removed, R4 reagent and R5 reagent were added to the magnetic particle, and chemiluminescence intensity was then measured. The measured chemiluminescence intensity was fitted to a calibration curve, to determine the level of each biomarker. The calibration curve was prepared with use of a calibrator of each biomarker. The calibrator was prepared from a recombinant protein of each biomarker.

### (6) Measured Results

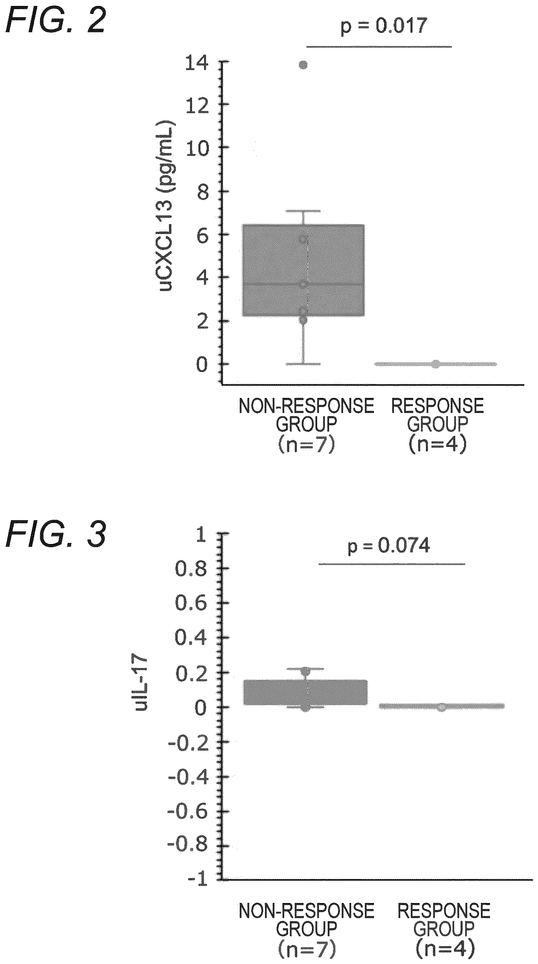

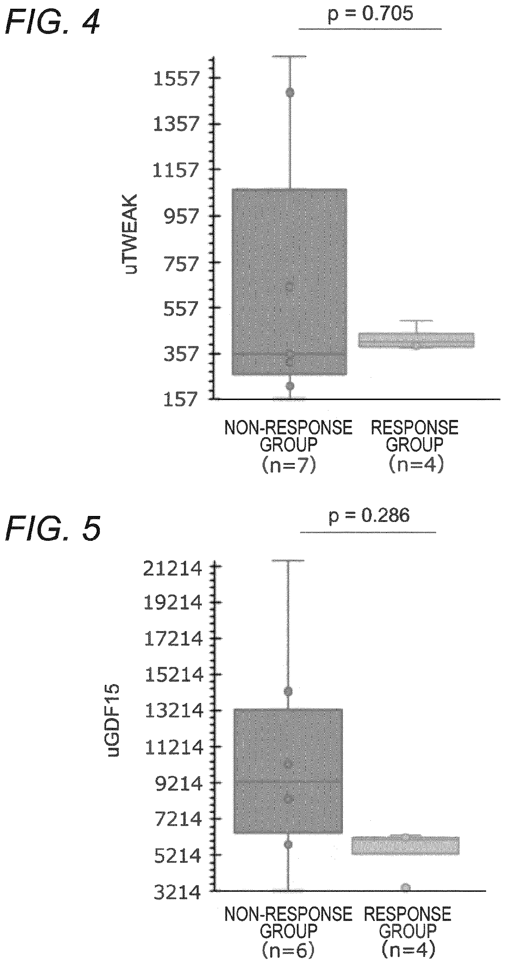

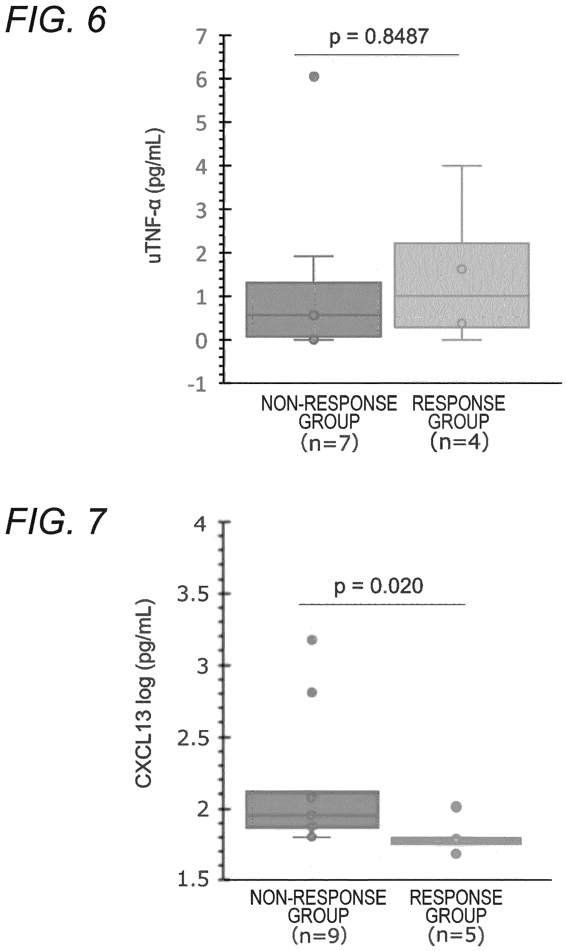

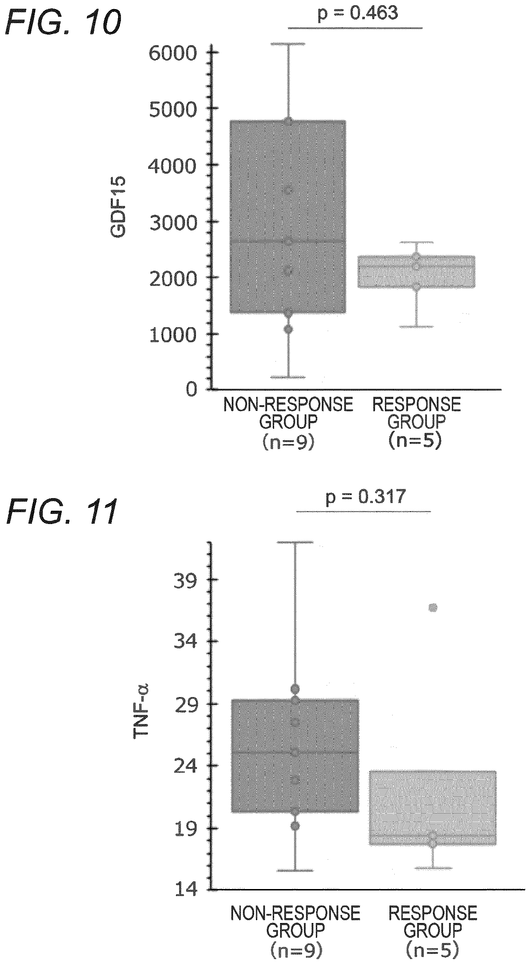

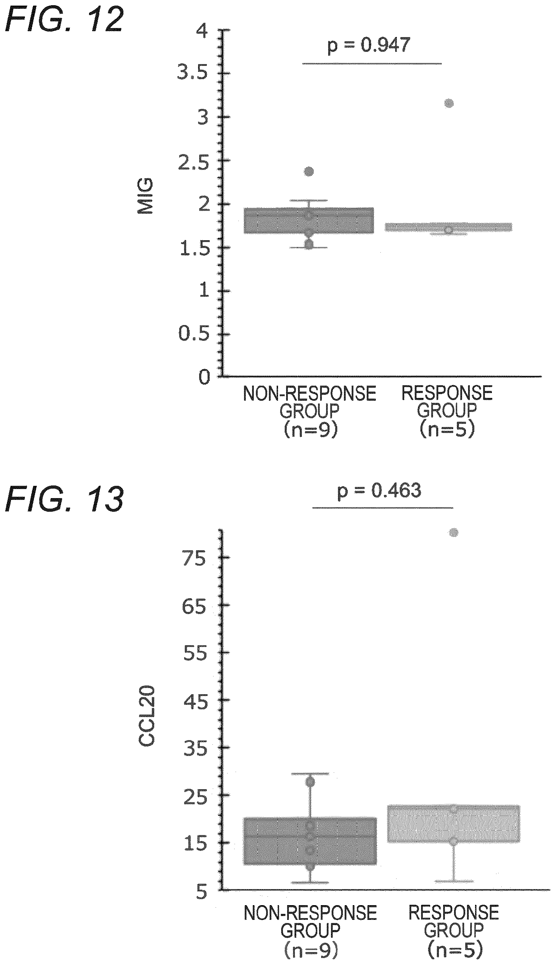

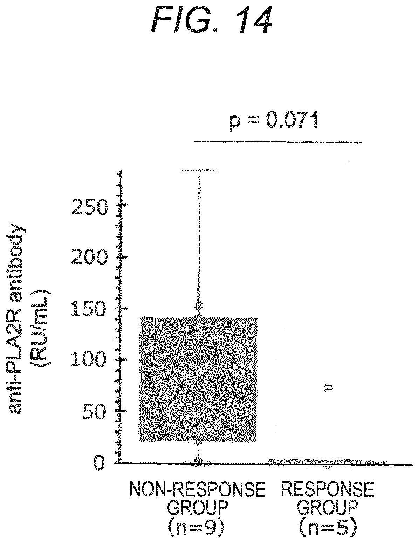

Levels of CXCL13, IL-17, TWEAK, GDF15, and TNF-α in the urine samples in the response group and the non-response group were illustrated in Figs. 2 to 6, respectively. Also levels of CXCL13, IL-17, TWEAK, GDF15, TNF-α, MIG, CCL20, and anti-PLA2R antibody in the blood samples in the response group and the non-response group were illustrated in Figs. 7 to 14, respectively. In each drawing, patients whose biomarkers could not be measured typically due to shortage of the sample volume were excluded. Referring to Fig. 2, CXCL13 levels of the urine samples in the response group were found to be significantly lower than those in the non-response group (p = 0.017). Referring to Fig. 7, also for the blood samples, CXCL13 levels in the response group were found to be significantly lower than those in the non-response group (p = 0.020). This taught that CXCL13 in urine and blood can be a biomarker that enables determination of the efficacy of chemotherapy with use of the anti-CD20 antibody drug for patients with membranous nephropathy. That is, the CXCL13 level in the urine sample or blood sample was proved to suggest the efficacy of chemotherapy with use of the anti-CD20 antibody drug for patients with membranous nephropathy. Figs. 2 and 7 also taught that a threshold value that properly distinguish the response group and the non-response group can be set for the CXCL13 level. It was therefore found that whether or not chemotherapy with use of the anti-CD20 antibody drug is effective for patients with membranous nephropathy can be determined, by comparing such threshold value with the CXCL13 level of the urine samples or blood samples.

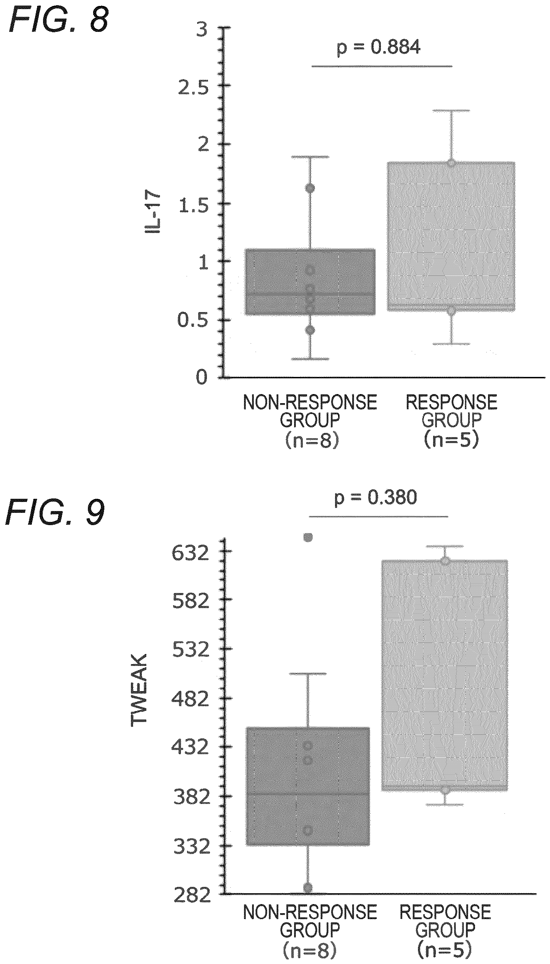

Referring to Figs. 3 to 6, all of IL-17, TWEAK, GDF15, and TNF-α in the urine samples were not found to be significantly different between the response group and the non-response group. Referring to Figs. 8 to 14, all of IL-17, TWEAK, GDF15, TNF-α, MIG, CCL20 and anti-PLA2R antibody in the blood samples were again not found to be significantly different between the response group and the non-response group. As described previously, these biomarkers have been reported to be associated with glomerulonephritis and/or autoimmune diseases. These biomarkers were, however, not found to associate with the efficacy of chemotherapy with use of the anti-CD20 antibody drug for patients with membranous nephropathy.

## Claims

1. A method for obtaining information on efficacy of chemotherapy for a patient with membranous nephropathy, the method comprising measuring CXCL13 in a biological sample collected from the patient with membranous nephropathy, wherein the efficacy of chemotherapy for the patient with use of an anti-CD20 antibody drug is suggested by a result of the measuring.

2. The method according to claim 1, wherein a measured value of CXCL13 below a threshold value suggests the efficacy of chemotherapy for the patient, and the measured value of CXCL13 equal to or above the threshold value suggests non-efficacy of chemotherapy for the patient.

3. The method according to claim 1, wherein the anti-CD20 antibody drug is at least one selected from the group consisting of rituximab, obinutuzumab, ofatumumab, ibritumomab tiuxetan, tositumomab, ublituximab and pertuzumab.

4. The method according to claim 1, wherein the biological sample is a blood sample or urine.

5. The method according to claim 1, wherein the chemotherapy uses other drug, besides the anti-CD20 antibody drug.

6. The method according to claim 5, wherein the other drug is at least one selected from the group consisting of immunosuppressant and steroid.

7. The method according to claim 6, wherein the immunosuppressant is a calcineurin inhibitor.

8. The method according to claim 7, wherein the calcineurin inhibitor is at least one selected from the group consisting of tacrolimus, pimecrolimus, cyclosporine, and voclosporin.

9. The method according to claim 6, wherein the steroid is corticosteroid.

10. A method for assisting determination of efficacy of chemotherapy for a patient with membranous nephropathy, the method comprising:
measuring CXCL13 in a biological sample collected from the patient with membranous nephropathy; and
determining, from a result of the measuring, the efficacy of chemotherapy for the patient with use of an anti-CD20 antibody drug.

11. The method according to claim 10, wherein the chemotherapy is determined to be effective on the patient if a measured value of CXCL13 is below a threshold value, and the chemotherapy is determined to be not effective on the patient if the measured value of CXCL13 is equal to or above the threshold value.
